# EUROPEAN PATENT SPECIFICATION

(11) **EP 1 886 655 B1**
(45) Date of publication and mention of the grant of the patent: **20.08.2014**
(21) Application number: 07013683.3
(22) Date of filing: 12.07.2007
(51) Int. Cl.: A61F 13/12, A61F 9/04

(54) **Disposable eye patch**
Einweg-Augenklappe
Pansement oculaire jetable

(30) Priority: 09.08.2006 US 500906
(43) Date of publication of application: 13.02.2008
(73) Proprietor: Glendale Protective Technologies, Inc., Smithfield RI 02917 (US)
(72) Inventor: McLear, Mark, Lincoln Rhode Island 02865 (US)
(74) Representative: Houghton, Mark Phillip

(56) References cited:
- EP-A2- 0 375 211
- US-A- 3 092 103
- US-A- 4 979 811
- US-A1- 2001 051 165
- US-A1- 2004 074 502
- US-B1- 6 320 094

## Description

Cosmetic surgical procedures (e.g., plastic surgery) and professional facial care procedures are becoming increasingly popular. In some cases, patients request such procedures for facial parts such as the nose bridge, forehead, temples, and areas immediately surrounding the eyes. During surgical or other procedures directed to such facial parts, doctors and other professionals often must have access to as much unobstructed area as possible. At the same time, the doctors or other professionals need to avoid damaging the patient's eyes by various surgical, medical and cosmetic items, such as abrasion systems, chemicals, air jets, air streams, liquids, medicines, medicine applicators, surgical tools (e.g., scalpels, hemostats, needles, etc.), light emitting devices and the light emitted therefrom, and other devices. The light emitting devices include lasers, intense pulsed light (IPL) devices, which emit pulses of intense diffuse light spanning a broad range of wavelengths, and light emitting diode (LED) devices, which emit light in a relatively narrow range of wavelengths.

U.S. Patent No. 6,320,094 to Arnold et al. discloses a disposable eye patch that allows doctors or other professionals to have full access to areas around the eyes for surgery and facial care procedures, and effectively protects a wearer's eyes during various types of procedures. US2001/051165A1 discloses an occlusive plaster consisting of at least one layer of a foam material which is provided on the lower side with a skin compatible self-adhesive layer. This plaster is intended to treat strabism and has no protecting purpose. No property of the multilayer structure is disclosed in this document. EP0375211A2 also discloses a light-occlusive eye patch for the treatment of strabism, without providing any indication as regards protection of the eye against light emissions. US6320094B1 discloses a protective eye patch which illustrates the technological background of the invention. US4979811A discloses an eye lid cover for protection of the eye during sun bathing. US2004/074502A1 discloses an eye shield without any light protecting layer, only keeping a patient's eyes closed during surgical procedure.

GPT Glendale, Inc. has marketed a disposable eye patch intended for use with IPL devices for over a year. The eye patch is similar to embodiments disclosed in the 094 patent, but includes a black vinyl layer having a thickness of 4 mil (0.102 mm). This eye patch effectively protects a wearer's eye from light emitted from an IPL device.

GPT Glendale, Inc. has also marketed for over a year a disposable eye patch intended for use with laser devices. The eye patch is similar to embodiments disclosed in the 094 patent, but includes a metallic layer. This eye patch effectively protects a wearer's eye from light emitted from a laser device.

The above-described eye patches are effective for their intended uses, and some are suitable for multiple types of use. For example, the IPL patch can also be used for microdermabrasion procedures, and the laser patch can also be used for IPL procedures, LED procedures and microdermabrasion procedures.

However, the IPL patch is not suitable for LED or laser procedures. While the laser patch is suitable for laser, IPL and LED procedures, it is more expensive to manufacture than the IPL patch, and is somewhat stiffer, and thus somewhat less comfortable to the wearer. It would be advantageous to provide a disposable eye patch that is less expensive to manufacture, and more comfortable to the wearer, while providing suitable protection against not only light from IPL devices, but also light from LED devices, and preferably also light from low-powered laser devices.

The present invention provides a patch as defined in claim 1. The patch system may include the features of any one or more of dependent claims 2 to 12.

Exemplary embodiments of this invention provide a small size disposable eye patch that allows doctors or other professionals full access to areas around the eyes for surgery and facial care procedures, including light procedures such as LED procedures, IPL procedures and preferably also low-powered laser procedures, and is comfortable for the patient to wear.

A disposable eye patch according to exemplary embodiments includes at least one sheet member and a light-occluding layer such as a vinyl layer attached to the sheet member. The eye patch may include at least two sheet members, with the light-occluding layer interposed between the sheet members. The eye patch preferably has adhesive on a peripheral portion of the eye patch for attaching the eye patch to a wearer's face.

These and other features and advantages are described in or are apparent from the following description of exemplary embodiments.

Embodiments of the invention will now be described with reference to the accompanying drawings, in which like numerals represent like parts, and in which:
Fig. 1 is a top plan view of a disposable eye patch according to a first exemplary embodiment of the invention;
Fig. 2 is a bottom plan view of the disposable eye patch of Fig. 1;
Fig. 3 is a cross-sectional view of the disposable eye patch of Fig. 1;
Fig. 4 is a top plan view of the disposable eye patch of Fig. 1 mounted on a release layer;
Fig. 5 is a cross-sectional view of the disposable eye patch and the release layer of Fig. 4;
Fig. 6 shows the disposable eye patch of Fig. 1 being used during a facial care procedure; and
Fig. 7 is a cross-sectional view of a disposable eye patch according to an embodiment not part of the present invention.

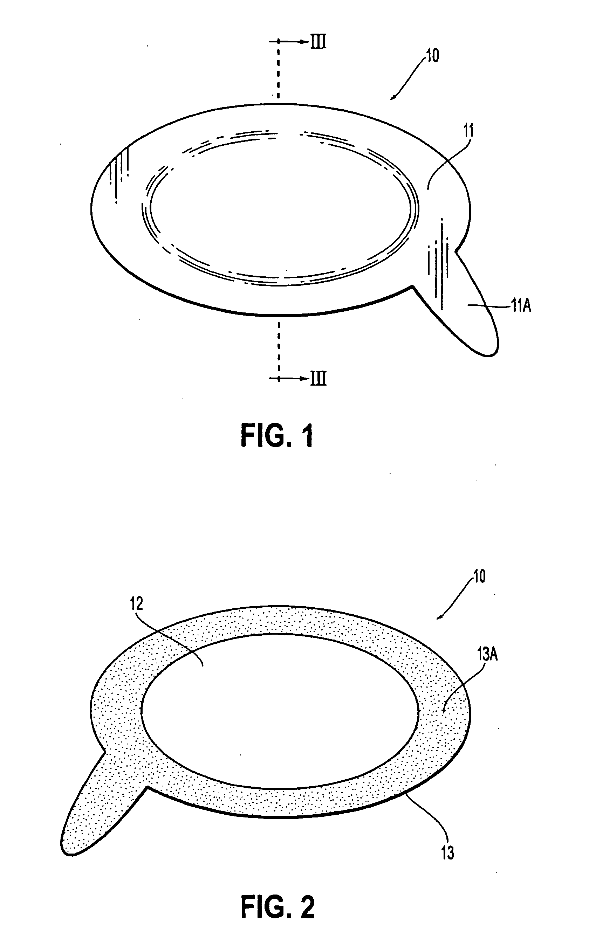

As shown in Figs. 1 and 2, a disposable eye patch 10 includes a first sheet member 11 and a second sheet member 12. These first and second sheet members are preferably oval in shape, although other shapes, such as a teardrop shape, polygonal shape, or the like, are also possible. "Oval" in the context of this application includes elliptical, oblong, and egg shapes.

As can be seen in Figs. 1 and 2, the plan-view shapes of the first sheet member 11 and the second sheet member 12 in the depicted embodiment include an eyeball-covering area that completely covers an eyeball. Although the eye patch 10 preferably has a generally oval shape, teardrop shape, or the like, a portion such as tab 11 A may be provided on an edge of the eye patch 10 to allow easy gripping and peeling of the eye patch 10 from the eye and eyelid after use. Such a tab should be large enough for fingers to grip, but is preferably as small as possible or otherwise configured to leave as much facial area exposed as possible.

The eye patch 10 includes a first adhesive layer 13 on one side of the first sheet member 11 for adhering the eye patch 10 onto the tissue surrounding the patient's eye and preferably also for adhering the first sheet member 11 to the second sheet member 12. This first adhesive layer 13 may, for example, have a plan-view size approximately equal to the plan-view size of the first sheet member 11 and is preferably made of a pressure-sensitive adhesive (i.e., it may acquire greater adhesion with an adjacent surface as pressure between that surface and the adhesive is increased), preferably a latex-free and hypoallergenic material. The adhesion of the adhesive layer 13 should be strong enough to adhere reliably to the skin but weak enough to be easily removed from the skin after use. The adhesive layer 13 may be directly applied to the first sheet member 11, e.g., by being sprayed on or applied by a roller, brush or the like, or may be a prefabricated sheet or ring that is, e.g., coated with adhesive on both sides. Such a prefabricated sheet or ring is attached to the first sheet member 11 by the adhesive on one side, and the adhesive on the other side adheres to the wearer's skin. The adhesive on one side may be the same as or different from the adhesive on the other side. Any other known or later developed form of adhesive layer is also acceptable as the adhesive layer 13.

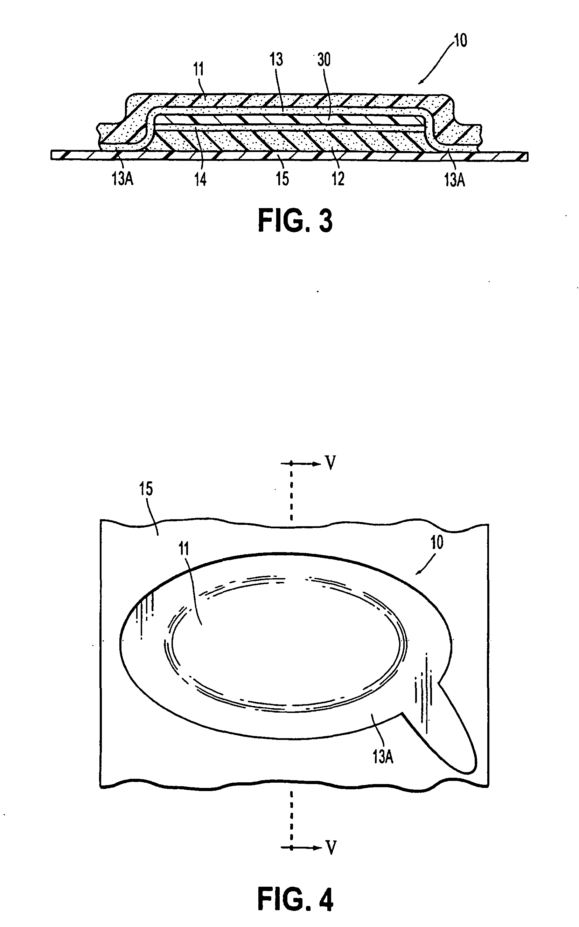

As shown in Fig. 3, a light-occluding layer 30 is located on one side of sheet member 11, preferably between the first sheet member 11 and the second sheet member 12 as shown. The light-occluding layer 30 may have an oval or other shape like that discussed above. Thus, the plan-view shape of the light-occluding layer 30 may include an eyeball-covering area that completely covers an eyeball. All of the light-occluding layer 30, or at least all of the eyeball-covering area of the light-occluding layer 30, prevents all light, or at least eye-damaging light, such as direct light from any, all or any combination of a low-powered laser device, an IPL device or an LED device, from reaching the eye. In some embodiments, the light-occluding layer 30 prevents at least eye-damaging light, and optionally all light, from reaching the eye by being free of holes or pores. In other embodiments, the light-occluding layer 30 prevents at least eye-damaging light, and optionally all light, from reaching the eye by having holes or pores that are tortuous, and may allow some light to pass, but only after the light has been reflected off of various surfaces within the holes or pores, and thus is not directly incident upon the eye. In other words, the tortuous characteristic of the holes or pores prevents light from passing straight through to the eye, and preferably causes any eye-damaging light that enters the holes or pores to be dispersed, such that it is no longer eye-damaging.

The light-occluding layer 30 may be smaller than the first sheet member 11, and may be positioned such that a peripheral portion 13A of the adhesive layer 13 is exposed. Optionally, the light-occluding layer 30 may be the same size as the first sheet member 11. In this case, an adhesive layer could be provided on a peripheral portion of the light-occluding layer 30 for adhering the eye patch 10 to the wearer's face.

In embodiments, the adhesive layer 13 may, but need not be, provided over the entire sheet member 11. For example, the adhesive layer 13 may be applied at intermittent locations on the sheet member, such as in spaced-apart spots or strips. If the light-occluding layer 30 is attached to the sheet member 11 by another method such as stitching, thermal bonding, or any other known or later developed attachment method, then it is acceptable to apply the adhesive only at the exposed periphery of the sheet member 11. The adhesive layer 13 may be on the entire periphery of the sheet member 11, or on only part of the periphery of the sheet member 11.

Furthermore, it should be appreciated that, when the light-occluding layer 30 is attached to the sheet member 11 by adhesive, the adhesive used to attach the light-occluding layer to the sheet member 11 may be the same as or different from the adhesive provided on the peripheral portion of the sheet member 11 for attaching the eye patch 10 a wearer's face. For example, the adhesive that contacts the wearer's face may be a hypoallergenic and/or pressure-sensitive and/or latex free adhesive as described above, while the adhesive between the light-occluding layer 30 and the sheet member 11 is an adhesive especially designed to promote good adhesion between the sheet member material, e.g., foamed plastic material, and the material of the light-occluding layer. In embodiments, two adhesives may be used -- one initially applied to the light-occluding layer 30 and one initially applied to the sheet member 11. In such embodiments, for example, the adhesion between these members may be enhanced over the adhesion between the sheet member 11 and the patient's tissue.

The second sheet member 12 preferably has a size equal to or slightly larger than the light-occluding layer 30. The second sheet member 12 also is preferably shaped (e.g., smaller than the first sheet member 11) so that a peripheral portion of the adhesive layer 13 remains exposed.

Like the sheet member 11, the sheet member 12 may be directly or indirectly attached to the light-occluding layer 30 by an adhesive layer 14, which may be initially provided on the second sheet member 12, on the light-occluding layer 30, and/or on the first sheet member 11. The second adhesive layer 14 may be of the same material as the first adhesive layer 13, or a different material. The adhesive layer 14 may be provided over the entire sheet member 12, light-occluding layer 30 and/or sheet member 11, or may be applied at intermittent locations on the sheet member 12, light-occluding layer 30 and/or sheet member 11, such as in spaced-apart spots or strips. It is also acceptable to attach the light-occluding layer 30 to the sheet member 11 directly or indirectly by a method other than using adhesive, such as stitching, thermal bonding, or any other known or later developed attachment method.

The first sheet member 11 has a size that sufficiently covers the patient's eye and eyelid when applied. For instance, for an adult patient, the first sheet member 11 preferably has a length of from about 40 mm to about 60 mm, more preferably about 50 mm, and a width of from about 20 mm to about 35 mm, more preferably about 28 mm. The second sheet member 12 preferably joins the first sheet member 11 at a central portion of the first adhesive layer 13 as shown in Fig. 2.

Having the above-described length and width allows the eye patch 10 to fit within the eye socket of a patient, and thus maximizes the facial area accessible to a doctor or other professional. The part of the eye patch 10 that contacts most of the patient's eyelid is preferably free of exposed adhesive. This is more comfortable to the wearer, and allows the eye and eyelid to slightly move underneath the eye patch 10. Preferably, the side of the sheet member 12 facing the eye and eyelid is free of adhesive, and may be comprised of a particularly non-adherent material. A biocompatible foamed plastic material is one example of a material that is particularly non-adherent. This also prevents needless pain or discomfort when the eye patch is removed after use. One example of a suitable biocompatible foamed plastic material is the product known as 9781, Foam Medical Tape, made by 3M, located in St. Paul, Minnesota.

A peripheral portion 13A of the first adhesive layer 13 that is not overlapped by the second sheet member 12 should be large enough to provide sufficient adhesion of the eye patch 10 to the area of the face surrounding the eye. For example, the peripheral portion 13A may have a width of approximately 5 mm. In the depicted embodiments, the peripheral portion 13A extends along an entire periphery of the sheet member 11, and the light-occluding layer 30 and the sheet member 12 do not overlap any part of the peripheral portion 13A. In other embodiments, the light-occluding layer 30 and/or the sheet member 12 may overlap part of the peripheral portion 13A.

The first sheet member 11 and the second sheet member 12 each preferably have a thickness in a range of from about 0.1 mm to about 5 mm, more preferably from about 0.1 mm to about 2 mm, and even more preferably about 0.5 mm. The appropriate thickness may vary depending upon the type of procedure for which the eye patch is intended to be used, but in general, a thinner eye patch is desired to reduce the bulkiness of the eye patch 10 and increase the comfort of the patient. For example, for a so-called microdermabrasion process, in which aluminum-oxide crystals or the like are discharged from a wand onto a patient's face, an eye patch 10 with a first sheet member 12 with a thickness of about 0.5 mm and a second sheet member 12 with a thickness of about 0.5 mm effectively protects the eye. The light-occluding layer 30 may also help physically protect the eye during such treatments.

The first sheet member 11 is preferably made of biocompatible foamed plastic material. The second sheet member 12 is also preferably made of biocompatible foamed plastic material. The material of the light-occluding layer 30 will be discussed below.

Various light procedures are becoming increasingly popular as facial and other procedures. For example, IPL procedures are used for wrinkle reduction treatment. LED procedures are used for wound healing and pain management. LEDs designed for such purposes provide localized heat to a cut, sprain or the like, and thereby facilitate the healing process. Laser procedures are used in surgery and in removal of birthmarks, age spots and tattoos. To protect the eye from light procedures such as LED procedures, IPL procedures and low-powered laser procedures, the eye patch 10 preferably achieves an L4 rating under any, all, or any combination of test conditions D, I, R and M for laser radiation in the wavelength range of 315-1400 nm under the European Standard EN 207, as in effect on January 1, 2006, including any amendments, annexes and the like that are in effect on that date. For reference, these test conditions are summarized in the following table, which shows the duration of a standard test for filters and eye protectors against laser radiation according to EN 207.

| Test Conditions For Laser Type | Typical Laser Type | Pulse length (seconds) | Number Of Pulses |
|---|---|---|---|
| D | Continuous wave laser | 10 | 1 |
| I | Pulsed laser | 10⁻⁴ to 10⁻¹ | 100 |
| R | Q switch pulsed laser | 10⁻⁹ to 10⁷ | 100 |
| M | Mode-coupled pulsed laser | <10⁻⁹ | 100 |

As known by those skilled in the art, the L4 rating is a scale number in a range of L1 to L10, and indicates the maximum spectral transmittance at the wavelength of the laser used for testing. Spectral transmittance refers to the amount of energy transmitted through a test specimen when irradiated by the laser used for testing. To achieve an L4 rating, as shown in EN 207, the eye patch must allow spectral transmittance of no greater than 10<-4> . As known by those skilled in the art, the unit of measurement of spectral transmittance is watts/m<2> . To achieve an L5 rating, the eye patch must allow spectral transmittance of no greater than 10<-5> ; to achieve an L6 rating, the eye patch must allow spectral transmittance of no greater than 10<-6> ; and so forth.

Other test conditions used to establish the L4 rating, such as laser beam diameters, and power and energy density of the laser beams at the various wavelength ranges, are set forth in EN 207, and are known to those skilled in the art.

For the eye patch 10 to achieve the L4 rating, the light-occluding layer 30 may, for example, be made of black opaque vinyl material with a thickness of 0.203 mm (8 ml) or more. One example of a suitable black vinyl material is the product known as FLEXmark V 800 F BLACK V-344 SPEC 50K-8, made by FLEXcon Company, Inc., located in Spencer, Massachusetts. In addition to a vinyl layer of 0.203 mm thickness, this product includes a pressure sensitive acrylic adhesive layer of 0.030-0.033 mm (1.2-1.3 mil) thickness. The adhesive layer may be used as one of the adhesive layers described above. Another example is the product known as FLEXmark V 800 F BLACK V-23 90 PFW, also made by FLEXcon Company, Inc. In addition to a vinyl layer of 0.203 mm (8 mil) thickness, this product includes a pressure sensitive acrylic adhesive layer of 0.043-0.044 mm (1.7-1.8 mil) thickness. The adhesive layer may be used as one of the adhesive layers described above. Some embodiments may include a metallic layer, but advantages of using a non-metallic layer such as vinyl material instead of a metal layer as the light-occluding layer 30 are that, for example, manufacturing costs are reduced, and the eye patch 10 may be slightly more flexible (and thus more comfortable to the wearer) than when a metal layer is used. In the context of this application, "non-metallic layer" means a layer that does not include metal as a primary component and/or as a primary support of the layer. For example, a layer that does not include any metal is a non-metallic layer. As another example, a layer that includes an amount of metal that is less than about 50%, or less than about 40%, or less than about 30%, or less than about 20%, or less than about 10%, or less than about 5%, or less than about 1% of the total weight, volume, and/or plan-view area of the layer, is also a non-metallic layer. In this latter example, the metal could be in the form of fibers, strips, flakes, particles or the like that are incorporated in and/or supported by a non-metallic material.

Various woven or knitted materials may also be suitable as the light-occluding layer, provided that they prevent eye-damaging light from reaching an eye, as discussed above.

Of the components of the eye patch 10, the light-occluding layer 30 is typically the primary contributor to resistance to light. However, because the test results may also vary depending on the material and thickness of the sheet members 11 and 12, testing to confirm the L4 rating is performed for the eye patch 10 as a whole.

The color of the eye patch 10 may be the natural color of the foamed plastic material, such as off-white, cream, or the like, or any other desired color such as beige, gray, black, fluorescent green, etc.

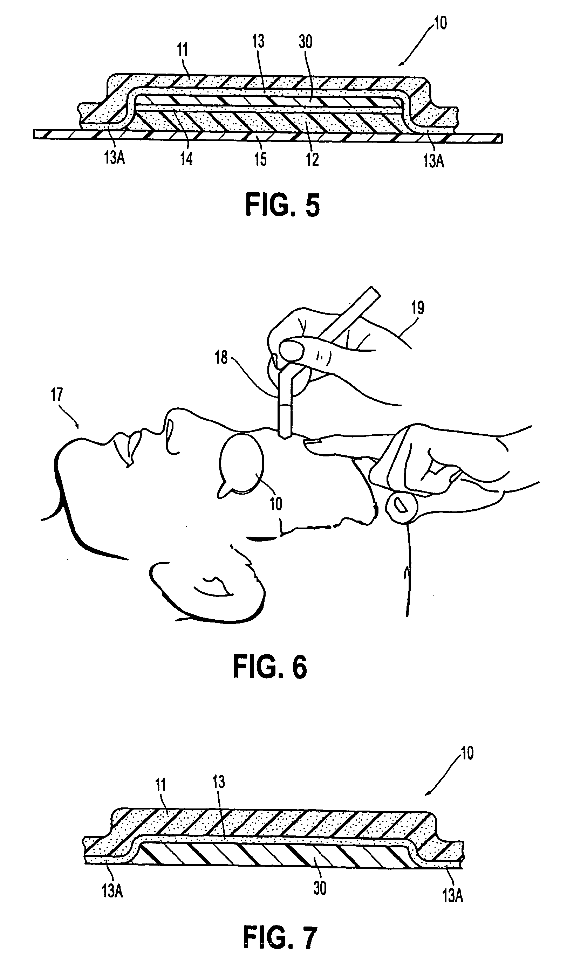

As shown in Figs. 4 and 5, to prevent the peripheral portion 13A of the adhesive layer 13 and the eye-contacting side of the second sheet member 12 of the eye patch 10 from being contaminated, the peripheral portion 13A of the adhesive layer 13 may be attached to a release layer 15, which is removed prior to use of the eye patch 10. The release layer 15 may, for example, be a waxed paper, plastic film or the like.

The adhesive layer 13 may be excluded from covering all or part of the tab 11A. Alternatively, all or part of the tab 11 A may include a non-stick layer (not shown) to restrict the tab 11A from adhering to the release layer 15 or to a wearer's face. This makes it easier for a user to grasp the tab 11A and remove the eye patch 10 from the release layer 15 or from the wearer's face.

When the eye patch 10 is to be applied to a patient, the release layer 15 is first peeled off from the disposable eye patch 10. When the release layer 15 has been removed, the adhesive is exposed. The eye patch 10 is then positioned over the patient's closed eye and eyelid, and the edge of the eye patch 10 is gently pressed to seal the eye patch to facial tissue surrounding the patient's eye and eyelid. After use, the eye patch 10 is gently peeled away from the eye and eyelid.

Fig. 6 shows an example of a patient 17 wearing a disposable eye patch 10. The tissue areas directly around the patient's eye are exposed and accessible to the doctor or therapist 19. While the patient's eyes are thus protected, the doctor or therapist performs a procedure using a tool 18. The procedure may be any of the procedures described above, including light procedures such as LED procedures, IPL procedures or low-powered laser procedures. In the context of this disclosure, "low-powered laser procedures" means procedures using lasers that output a beam having an energy of not greater than about 5 watts/cm<2> at the point at which the beam strikes the eye patch. The above-described embodiment in which the light-occluding layer 30 is made of 8 mil black opaque vinyl material is suitable for use with lasers that output a beam having an energy of about 1 watt/cm<2> at the point at which the beam strikes the eye patch. In embodiments, the eye patch 10 should be sufficient to stop direct laser radiation from a 1 watt/cm<2> system (i.e., a system that outputs a beam having an energy of about 1 watt/cm<2> at the point at which the beam strikes the eye patch) for at least 10 seconds. In the context of this patent application, "stop direct laser radiation" means "maintain the rating of L4 during direct laser radiation." Various embodiments may be sufficient to stop direct laser radiation from a laser having an energy of about 2 watts/cm<2> at the point at which the beam strikes the eye patch for at least 10 seconds, or from a laser having an energy of about 3 watts/cm<2> at the point at which the beam strikes the eye patch for at least 10 seconds, or from a laser having an energy of about 4 watts/cm<2> at the point at which the beam strikes the eye patch for at least 10 seconds, or from a laser having an energy of about 5 watts/cm<2> at the point at which the beam strikes the eye patch for at least 10 seconds. If the laser power is increased, the material and/or thickness of the light-occluding layer 30 should be selected accordingly to ensure that the eye patch 10 will stop direct laser radiation for at least 10 seconds. One of ordinary skill in the art could readily make such selections without undue experimentation in light of the present disclosure and the knowledge of those of ordinary skill in the art.

Fig. 7 is a cross-sectional view of a disposable eye patch 10. As shown in Fig. 7, the eye patch 10 includes sheet member 11 and light-occluding layer 30, both of which may have an oval or other shape like that discussed above. The sheet member 11 may be sized as discussed above. One side of the light-occluding layer 30 may be adhered to the sheet member 11 via adhesive layer 13. The eye patch in this embodiment does not include a second sheet member on the other side of the light-occluding layer 30.

While the invention has been described in conjunction with specific embodiments described above, these embodiments are illustrative and not limiting. Various substitutes, modifications and improvements are possible within the scope of the invention.

## Claims

1. A disposable eye patch, comprising:
a first sheet member (11);
an adhesive layer (13) attached to the first sheet member, the adhesive layer on a part of or the entire periphery of the first sheet member ;
a non-metallic light-occluding layer (30) attached to the first sheet member such that a peripheral portion of the adhesive layer is exposed, the eye patch achieving a rating of L4 for at least one of the test conditions D, I, R or M, according to European Standard EN 207 as in effect on January 1, 2006, and maintaining the L4 rating during direct laser radiation for at least 10 seconds the light-occluding layer comprising a vinyl material having a thickness of at least 0.203 mm (8 mil); and
a second sheet member (12) attached to the light-occluding layer, the light-occluding layer being between the first and second sheet members;
the light-occluding layer and the second sheet member not overlapping any part of the peripheral portion of the adhesive layer.

2. The disposable eye patch according to claim 1, wherein the eye patch achieves a rating of L4 for at least the test conditions D, I and R according to said European Standard EN 207.

3. The disposable eye patch according to claim 1, wherein the first sheet member (11) comprises an eye-covering portion that is sized to fit entirely inside a human eye socket in at least one dimension of the eye socket.

4. The disposable eye patch according to claim 3, wherein the eye-covering portion (11) is sized to fit entirely inside the eye socket in both a lateral dimension of the eye socket and a vertical dimension of the eye socket.

5. The disposable eye patch according to claim 3, wherein the eye-covering portion (11) has a lateral dimension of from 40 mm to 60 mm.

6. The disposable eye patch according to claim 3, wherein the eye-covering portion (11) has a vertical dimension of from 20 mm to 35 mm.

7. The disposable eye patch according to claim 1, wherein the light-occluding layer (11) has a plan-view shape, the plan-view shape including an eyeball-covering area that completely covers an eyeball, all of the eyeball-covering area preventing eye-damaging light from reaching an eye.

8. The disposable eye patch according to claim 7, wherein all of the eyeball-covering area prevents all light from being directly incident upon the eye.

9. The disposable eye patch according to claim 1, wherein the first sheet member (11) and the second sheet member (12) are made of a same material.

10. The disposable eye patch according to claim 1, wherein the first (11) and second (12) sheet members each have a thickness in a range of from 0.1 mm to 5 mm.

11. The disposable eye patch according to claim 1, wherein at least one of the first sheet member (11) and the second sheet member (12) comprises biocompatible foamed plastic material.

12. The disposable eye patch according to claim 1, further comprising a tab (11A) provided at an edge of the first sheet member, the tab facilitating gripping of the eye patch.

## Patentansprüche

1. Einweg-Augenklappe, umfassend:
ein erstes Flächenstück (11);
eine an dem ersten Flächenstück befestigte Klebeschicht (13), wobei sich die Klebeschicht auf einem Teil des Umfangs oder auf dem gesamten Umfang des ersten Flächenstücks befindet;
eine nicht-metallische lichtausschließende Schicht (30), die an dem ersten Flächenstück befestigt ist, so dass ein peripherer Abschnitt der Klebeschicht freiliegt, wobei die Augenklappe eine Bewertung von L4 für zumindest eine der Testbedingungen D, I, R oder M nach der am 1. Januar 2006 wirksamen Europäischen Norm EN 207 erzielt, und die Bewertung L4 bei direkter Laserbestrahlung für mindestens 10 Sekunden beibehält,
wobei die lichtausschließende Schicht ein Vinylmaterial mit einer Dicke von mindestens 0,203 mm (8 mil) umfasst; und
ein zweites Flächenstück (12), das an der lichtausschließenden Schicht befestigt ist, wobei sich die lichtausschließende Schicht zwischen den ersten und
zweiten Flächenstücken befindet;
wobei sich die lichtausschließende Schicht und das zweite Flächenstück auf keinem Teil des peripheren Abschnitts der Klebeschicht überlappen.

2. Einweg-Augenklappe nach Anspruch 1, worin die Augenklappe eine Bewertung von L4 zumindest für die Testbedingungen D, I und R nach der besagten Europäischen Norm EN 207 erzielt.

3. Einweg-Augenklappe nach Anspruch 1, worin das erste Flächenstück (11) einen augenabdeckenden Abschnitt umfasst, der eine solche Größe aufweist, dass er völlig in eine menschliche Augenhöhle in zumindest einer Dimension der Augenhöhle passt.

4. Einweg-Augenklappe nach Anspruch 3, worin der augenabdeckende Abschnitt (11) eine solche Größe aufweist, dass er sowohl in einer lateralen Dimension der Augenhöhle als auch in einer vertikalen Dimension der Augenhöhle völlig in die Augenhöhle passt.

5. Einweg-Augenklappe nach Anspruch 3, worin der augenabdeckende Abschnitt (11) eine laterale Dimension von 40 mm bis 60 mm aufweist.

6. Einweg-Augenklappe nach Anspruch 3, worin der augenabdeckende Abschnitt (11) eine vertikale Dimension von 20 mm bis 35 mm aufweist.

7. Einweg-Augenklappe nach Anspruch 1, worin die lichtausschließende Schicht (11) eine Form in Draufsicht aufweist, wobei die Form in Draufsicht eine den Augapfel abdeckende Fläche aufweist, die einen Augapfel vollständig abdeckt, wobei die gesamte den Augapfel abdeckende Fläche verhindert, dass augenschädigendes Licht ein Auge erreicht.

8. Einweg-Augenklappe nach Anspruch 7, worin die gesamte den Augapfel abdeckende Fläche verhindert, dass Licht direkt auf das Auge auftrifft.

9. Einweg-Augenklappe nach Anspruch 1, worin das erste Flächenstück (11) und das zweite Flächenstück (12) aus einem selben Material gefertigt sind.

10. Einweg-Augenklappe nach Anspruch 1, worin die ersten (11) und zweiten (12) Flächenstücke jeweils eine Dicke in einem Bereich von 0,1 mm bis 5 mm aufweisen.

11. Einweg-Augenklappe nach Anspruch 1, worin zumindest das erste Flächenstück (11) und/oder das zweite Flächenstück (12) ein biokompatibles geschäumtes Kunststoffmaterial umfassen.

12. Einweg-Augenklappe nach Anspruch 1, ferner umfassend eine Lasche (11A), die an einem Rand des ersten Flächenstücks vorgesehen ist, wobei die Lasche das Ergreifen der Augenklappe erleichtert.

## Revendications

1. Pansement oculaire jetable, comprenant :
un premier organe de feuille (11) ;
une couche d'adhésif (13) attachée au premier organe de feuille, la couche d'adhésif étant disposée sur une partie ou sur la totalité de la périphérie du premier organe de feuille ;
une couche non métallique occultant la lumière (30) attachée au premier organe de feuille de telle sorte qu'une partie périphérique de la couche d'adhésif soit exposée, le pansement oculaire ayant un niveau de protection L4 pour au moins l'une des conditions d'essai D, I, R ou M conformément à la norme européenne EN 207 en vigueur le 1^{er} janvier 2006 et maintenant le niveau L4 sous un rayonnement laser direct pendant au moins 10 secondes, la couche occultant la lumière comprenant un matériau à base de vinyle ayant une épaisseur d'au moins 0,203 mm (8 mil) ; et
un deuxième organe de feuille (12) attaché à la couche occultant la lumière, la couche occultant la lumière étant disposée entre le premier et le deuxième organe de feuille ;
la couche occultant la lumière et le deuxième organe de feuille ne chevauchant aucune partie de la partie périphérique de la couche d'adhésif.

2. Pansement oculaire jetable selon la revendication 1, dans lequel le pansement oculaire a un niveau de protection L4 pour au moins les conditions d'essai D, I et R conformément à ladite norme européenne EN 207.

3. Pansement oculaire jetable selon la revendication 1, dans lequel le premier organe de feuille (11) comprend une partie couvrant l'oeil qui est dimensionnée de manière à s'ajuster entièrement à l'intérieur de l'orbite de l'oeil humain dans au moins une dimension de l'orbite de l'oeil.

4. Pansement oculaire jetable selon la revendication 3, dans lequel la partie couvrant l'oeil (11) est dimensionnée de manière à s'ajuster entièrement à l'intérieur de l'orbite de l'oeil à la fois dans une dimension latérale de l'orbite de l'oeil et dans une dimension verticale de l'orbite de l'oeil.

5. Pansement oculaire jetable selon la revendication 3, dans lequel la partie couvrant l'oeil (11) a une dimension latérale de 40 mm à 60 mm.

6. Pansement oculaire jetable selon la revendication 3, dans lequel la partie couvrant l'oeil (11) a une dimension verticale de 20 mm à 35 mm.

7. Pansement oculaire jetable selon la revendication 1, dans lequel la couche occultant la lumière (11) a une forme vue en plan, la forme vue en plan comportant une zone couvrant le globe oculaire qui couvre complètement le globe oculaire, toute la zone couvrant le globe oculaire empêchant que de la lumière endommageant l'oeil n'atteigne l'oeil.

8. Pansement oculaire jetable selon la revendication 7, dans lequel la totalité de la zone couvrant le globe oculaire empêche toute la lumière de parvenir directement sur l'oeil.

9. Pansement oculaire jetable selon la revendication 1, dans lequel le premier organe de feuille (11) et le deuxième organe de feuille (12) sont faits du même matériau.

10. Pansement oculaire jetable selon la revendication 1, dans lequel le premier (11) et le deuxième (12) organe de feuille ont chacun une épaisseur comprise entre 0,1 mm et 5 mm.

11. Pansement oculaire jetable selon la revendication 1, dans lequel au moins l'un du premier organe de feuille (11) et du deuxième organe de feuille (12) comprend un matériau plastique moussé biocompatible.

12. Pansement oculaire jetable selon la revendication 1, comprenant en outre une languette (11A) prévue au niveau d'un bord du premier organe de feuille, la languette facilitant la saisie du pansement oculaire.
